Europäisches Patentamt

(19) European Patent Office

Office européen des brevets

(11) Publication number: **0 310 224 B1**

(12) **EUROPEAN PATENT SPECIFICATION**

(45) Date of publication of patent specification:
24.07.91 Bulletin 91/30

(51) Int. Cl.⁵: **A61M 25/00**

(21) Application number: **88306853.8**

(22) Date of filing: **26.07.88**

(54) **Catheter and sheath assembly.**

(30) Priority: **15.09.87 GB 8721637**

(43) Date of publication of application:
**05.04.89 Bulletin 89/14**

(45) Publication of the grant of the patent:
**24.07.91 Bulletin 91/30**

(84) Designated Contracting States:
**AT BE CH DE ES FR GR IT LI LU NL SE**

(56) References cited:
**GB-A- 2 113 095**
**GB-A- 2 118 840**
**US-A- 4 168 699**
**US-A- 4 244 370**

(73) Proprietor: **H.G. Wallace Limited**
**Whitehall Road**
**Colchester Essex CO2 8JH (GB)**

(72) Inventor: **Wallace, Henry George**
**Whitehall Road**
**Colchester Essex CO2 8JH (GB)**

(74) Representative: **Stebbing, Peter John Hunter**
**Riches, Mill Lane**
**Bradfield Manningtree Essex CO11 2QP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a catheter and sheath (or cannula) assembly particularly, although not exclusively, for use in medical practice.

Assemblies of a catheter sliding within a sheath are known for a number of applications. One of the difficulties which arises in the use thereof is that when the distal tip of the sheath has been urged into an opening, it is no longer possible to ascertain the precise depth of penetration of either the sheath or, in use, the catheter sliding within.

In vitro fertilisation has been an established medical technique since 1978. An essential device used in placing embryos into the uterus of a recipient is a catheter which can be utilised to enter the cervical canal per vaginum and to deposit the embryos into the uterus without damage. Such a catheter needs to utilise a soft and flexible synthetic polymer in order to avoid trauma to the cervical canal ; this of course presents problems in directing the catheter via the cervical canal.

This problem has, in part, been overcome by the utilisation of a catheter having a generally rigid outer sheath which is provided so that during the initial direction of the catheter to the external os of the cervical canal, the sheath can be moved to protect the distal tip of the catheter. The catheter can therefore be carried by the sheath through the cervical canal as far as the internal cervical os whereupon the catheter can be moved into the uterine cavity. The catheter can then be advanced and the sheath retracted.

It has been found in use that the performance of this device can be improved if it can be readily ascertained, with the distal end of the sheath in situ, where the distal end of the catheter will be.

In US-A-4224370 there is disclosed a catheter and sheath assembly for the implantation of urological prosthetic devices. This comprises a sheath having a plurality of visible gradations on its surface, and two spaced detents on the surface of the catheter each adapted for interengagement with a spring-biased plunger on the cannula in use. In use only one of these detents is visible.

GB-A-21143095 discloses a catheter and sheath assembly wherein the catheter is provided with a metric scale along its length so that the degree of insertion of the catheter can be checked. There are no gradations on the sheath.

US-A-4168699 similarly provides a catheter and sheath assembly wherein the sheath is provided with a scale along its length to determine penetration ; there are no gradations on the catheter.

GB-A-2118840 provides an assembly of a catheter and a sheath therefor suitable for use in in vitro fertilisation, the catheter being provided with a hub remote from its distal tip ; said catheter being formed of a soft flexible material and the sheath being substantially rigid, wherein the catheter is slidable relative to the sheath from a position in which the distal tips of the sheath and catheter are generally coincident to a position in which the distal tip of the catheter projects beyond said sheath.

The present invention is characterised in that the catheter is provided with visible gradations disposed on the surface of the catheter adjacent its hub, and in that the sheath is provided with visible gradations adjacent its distal tip.

Each of the sheath and catheter are preferably provided adjacent their first ends with hubs, said hubs being interlockable in a closed position such that the catheter is in its position of maximum extension. The catheter is soft and flexible and is provided with a terminal or side outlet constituted to limit trauma both externally to the uterine tissue, and internally to any embryos passing through the catheter. To this end it is preferred that the sheath is formed of a self-lubricating material such as "Teflon".

The invention will now be described, by way of illustration only, with reference to the accompanying drawings wherein :

Figure 1 shows a side view of the sheath/catheter assembly in its closed position,
Figure 2 shows a side view of the sheath,
Figure 3 shows a side view of the catheter,
Figure 4 shows a side view of the sheath and catheter assembly with the catheter withdrawn one graduation, and
Figure 5 shows a vertical cross section through a catheter tip.

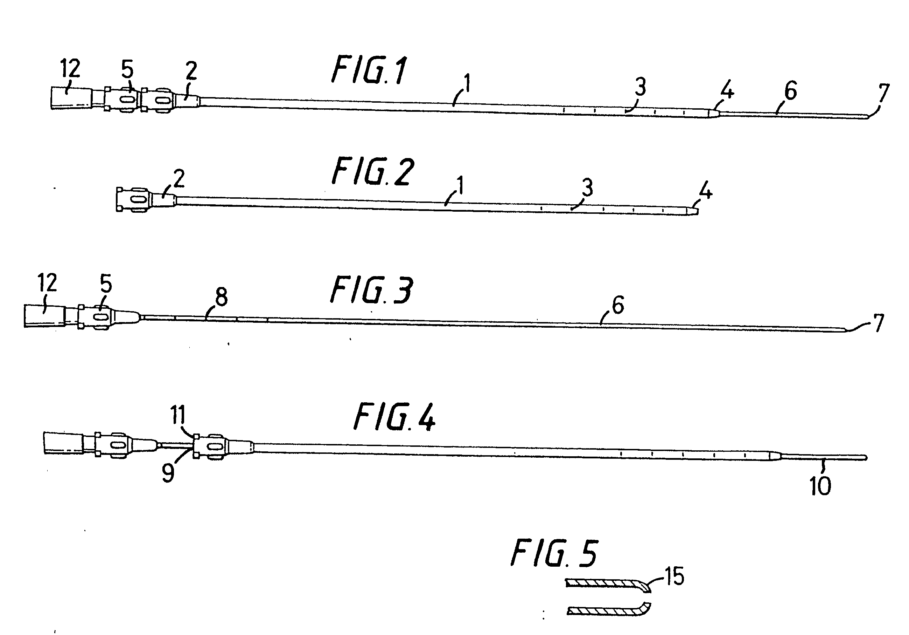

With reference to Figures 1 to 4 there is provided a sheath (1) being in the form of a hollow tube and provided towards its first end with a hub (2) provided with a series of axially extending fins to assist in manipulation. The distal tip of the sheath (1) is provided at intervals of one centimetre with gradations (3) which are marked as a plurality of rings about the length of the sheath. The gradations (3) may be slightly indented into the outer surface thereof. The remote distal tip (4) of the sheath (1) is chamfered for minimal trauma.

The sheath (1) is adapted to accommodate therewithin a catheter (6) having an external diameter such as to be an easy sliding fit within the sheath (1) and an internal diameter of a size to readily accommodate an embryo. The catheter (6) is provided at its one end with a hub (5) provided about its external surface with a plurality of axially extending fins to assist manipulation. The hub (5) is closed by a venting plug (12) which can be replaceably removed in order to gain access to the bore of the catheter (6). The catheter (6) is provided adjacent the hub (5) with a plurality of equally spaced gradations (8) which are clearly marked in a distinctive colour, and which may be slightly indented into the surface of the material of the catheter. The hubs (2) and (5) are adapted such that they interlock in their closed position as shown in Figure 1. This is done by arranging an interlocking annulus between the said hubs.

The distal tip (7) of the catheter (6) is shown in Figure 5. This is provided with a smoothly radiused chamfer (15) adapted to cause minimum trauma to the uterine tissues.

In use, the hubs (2) and (5) are disengaged and the sheath (1) is moved so that the distal tips of the catheter and sheath are co-incident, at which point the gradations on the catheter indicate zero. The sheath, which is formed of a generally rigid self-lubricating plastics material such as "Teflon" is advanced toward the external os of the cervical canal. With the distal end (4) of the sheath (1) positioned at the external os of the cervical canal, ideally the catheter (6) can be advanced so as to negotiate the cervical canal to the internal os and thence into the uterus. However, often an obstruction or tortuous path is encountered in the cervical canal. In this instance the catheter (6) may be gently rotated within the sheath (1) to assist its advance.

However, since the catheter is soft, the advance of the distal tip (7) thereof can be readily obturated. In this instance the sheath (1) is gently advanced through the cervical canal to overcome the obstruction or tortuous route so that the catheter (6) can pass into the uterus. When the sheath is also advanced into the cervical canal, its penetration is indicated by the gradations, and is added to the catheter penetration to give the position of the catheter tip.

It is important that the sheath does not penetrate beyond the internal os and for this reason the gradations toward its distal tip may be inspected to ensure that this does not occur. With the distal tip of the catheter at the internal os of the uterus it is then possible to retract the sheath (1) while leaving the catheter in situ. This is a very delicate operation which is rendered facile by the gradations which are required so that it can be ensured that the retraction of the sheath is not associated with a commensurate retraction of the catheter. When the catheter is in situ and the sheath has been fully withdrawn the hubs (2) and (5) can be interlocked prior to the introduction of an embryo.

In this particular embodiment the catheter has a softness of approximately Durometer A scale 80 with a constant lumen of, for example, 0.75 (0.03 inches).

The terminal outlet (7) of the catheter (6) is shown in Figure 5 as being axial. The outlet (7) may, however, be peripheral so long as it is sized and shaped to cause minimum trauma to embryos.

The catheter (6) in accordance with this embodiment of the invention is preferably formed of a flexible synthetic polymer, non-toxic to human embryos, sperm and oocytes as far as is possible.

The present invention provides, therefore, an assembly of a catheter and sheath for use in in vitro fertilisation.

## Claims

1. An assembly of a catheter (6) and a sheath (1) therefor suitable for use in in-vitro fertilisation, the catheter being provided with a hub (5) remote from its distal tip (7) ; said catheter being formed of a soft flexible material and the sheath (1) being substantially rigid, wherein the catheter is slidable relative to the sheath from a position in which the distal ends of the sheath (4) and the catheter (7) are generally coincident to a position in which the distal end of the catheter projects beyond said sheath ; characterised in that the catheter is provided with visible gradations (8) disposed on the surface of the catheter adjacent its hub (5) and in that the sheath (1) is provided with visible gradations (3) adjacent its distal tip (4).

2. An assembly according to Claim 1 characterised in that each of the catheter and sheath are provided adjacent their first ends with hubs (2, 5), said hubs being interlockable in a closed position so that the distal tip of the catheter (7) is in its position of maximum extension.

3. An assembly according to either preceding Claim characterised in that the catheter is provided with a chamfered outlet (15) at its distal tip (7), or with a side outlet adjacent said distal tip (7).

4. A method of in-vitro fertilisation which comprises charging an assembly of a catheter and sheath therefor as claimed in Claim 1 with a fertilised ova, inserting the distal tip (4) of the sheath (1) into the cervical canal by a predetermined amount, and sliding the catheter (6) relative to the sheath by a desired amount to position the distal tip (7) of the catheter at the delivery site, and subsequently causing the fertilised ova to be discharged into the uterus.

## Patentansprüche

1. Anordnung aus einem Katheter (6) und einer Hülle (1) hierfür, die zur Verwendung bei der in-vitro-Befruchtung geeignet ist, wobei der Katheter mit einer Nabe (5) von seinem distalen Ende (7) entfernt versehen ist, der Katheter aus einer weichen, flexiblen Material ausgebildet ist und die Hülle (1) im wesentlichen starr ist, und wobei der Katheter relativ zu der Hülle ausgehend von einer Position, in der die distalen Enden (4, 7) der Hülle und des Katheters im allgemeinen übereinstimmen, zu einer Position verschiebbar ist, in der das distale Ende des Katheters über die Hülle hinaus vorsteht, dadurch **gekennzeichnet,** daß der Katheter mit sichtbaren Abstufungen (8) versehen ist, die auf der Oberfläche des Katheters in der Nähe seiner Nabe (5) angeordnet sind, und daß die Hülle (1) mit sichtbaren Abstufungen (3) in der Nähe ihres distalen Endes (4) versehen ist.

2. Anordnung nach Anspruch 1, dadurch

gekennzeichnet, daß der Katheter und die Hülle jeweils in der Nähe ihrer ersten Enden mit Naben (2, 5) versehen sind, und die Naben in einer Schließstellung derart verriegelbar sind, daß das distale Ende (7) des Katheters in seiner maximal ausgefahrenen Position ist.

3. Anordnung nach einem der vorangehenden Ansprüche, dadurch gekennzeichnet, daß der Katheter mit einem abgerundeten Auslaß (15) am distalen Ende (7) oder mit einem Seitenauslaß in der Nähe des distalen Endes (7) versehen ist.

4. Verfahren zur in-vitro-Befruchtung, bei dem eine Anordnung aus einem Katheter und einer Hülle hierfür nach Anspruch 1 mit einer befruchteten Eizelle beschickt wird, das distale Ende (4) der Hülle (1) in den Gebärmutterhalskanal um eine vorbestimmte Größe eingeführt wird, und der Katheter (6) relativ zu der Hülle um eine gewünschte Größe verschoben wird, um das distale Ende (7) des Katheters an der Ausgabeseite zu positionieren, und bei dem schließlich bewirkt wird, daß die befruchtete Eizelle in den Uterus ausgegeben wird.

**Revendications**

1. Ensemble cathéter (6) et manchon (1) adapté à l'utilisation pour une fécondation in vitro, le cathéter étant doté d'un moyeu (5) loin de son extrémité distale (7) ; ledit cathéter étant formé d'un matériau flexible souple et le manchon (1) étant substantiellement rigide, dans lequel le cathéter peut coulisser relativement au manchon d'une position à laquelle les extrémités distales du manchon (4) et du cathéter (7) coïncident généralement à une position à laquelle l'extrémité distale du cathéter se projette au-delà dudit manchon ; caractérisé en ce que le cathéter est doté de graduations visibles (8) disposées sur la surface du cathéter, adjacentes à son moyeu (5), et en ce que le manchon (1) est doté de graduations visibles (3) adjacentes à son extrémité distale (4).

2. Ensemble selon la revendication 1, caractérisé en ce que le cathéter et le manchon sont dotés, adjacents à leur première extrémité, de moyeux (2, 5), lesdits moyeux pouvant être mutuellement verrouillés dans une position fermée de manière que l'extrémité distale (7) du cathéter se trouve dans sa position d'extension maximale.

3. Ensemble selon l'une quelconque des revendications précédentes, caractérisé en ce que le cathéter est doté d'un orifice de sortie chanfreiné (15) à son extrémité distale (7), ou d'un orifice de sortie latéral, adjacent à ladite extrémité distale (7).

4. Procédé de fécondation in vitro qui comprend les opérations consistant à charger un ensemble cathéter et manchon selon la revendication 1 d'ovules fécondés, à insérer l'extrémité distale (4) du manchon (1) dans le col de l'utérus d'une certaine profondeur, et à faire coulisser le cathéter (6) relativement au manchon sur une certaine distance afin de positionner l'extrémité distale (7) du cathéter à l'endroit de dépôt souhaité, et à décharger ensuite les ovules fécondés dans l'utérus.

EP 0 310 224 B1

FIG.1

12   5   2                    1                3        4    6        7

FIG.2

2              1            3            4

FIG.3

12   5        8                        6        7

FIG.4

11                                              10
9

FIG.5

15